Europäisches Patentamt

European Patent Office

Office européen des brevets

(19)

(11)  EP 1 002 528 B1

(12)  **FASCICULE DE BREVET EUROPEEN**

(45)  Date de publication et mention
de la délivrance du brevet:
**16.06.2004  Bulletin 2004/25**

(51)  Int Cl.⁷: $A61K\ 7/48$

(21)  Numéro de dépôt: **99402394.3**

(22)  Date de dépôt: **30.09.1999**

(54)  **Composition cosmétique sans transfert comprenant une dispersion de particules de polymère et un agent rhéologique particulier**

Nichtübertragende kosmetische Zusammensetzung die eine Dispersion von Polymerteilchen und ein spezifisches rheologiekontrollierenden Zusatzmittel enthält

Non-transfer cosmetic composition comprising a dispersion of polymer particles and a specific rheological agent

(84)  Etats contractants désignés:
**AT BE CH CY DE DK ES FI FR GB GR IE IT LI LU MC NL PT SE**

(30)  Priorité:  **09.11.1998  FR 9814076**

(43)  Date de publication de la demande:
**24.05.2000  Bulletin 2000/21**

(73)  Titulaire: **L'OREAL**
**75008 Paris (FR)**

(72)  Inventeur: **De La Poterie, Valérie**
**77820 Le Chatelet en Brie (FR)**

(74)  Mandataire: **Lhoste, Catherine**
**L'OREAL - D.I.P.I.**
**25-29 Quai Aulagnier**
**92600 Asnières (FR)**

(56)  Documents cités:
**EP-A- 0 749 746          EP-A- 0 749 747**
**WO-A-94/12190          WO-A-98/38981**
**WO-A-98/42298**

**Description**

La présente invention a trait à une composition contenant un polymère dispersible dans une phase grasse, destinée en particulier aux domaines cosmétique, dermatologique, pharmaceutique et hygiénique. Plus spécialement, l'invention se rapporte à une composition sans transfert pour le soin et/ou le maquillage de la peau aussi bien du visage que du corps humain, des muqueuses comme les lèvres et l'intérieur des paupières inférieures, ou encore des phanères comme les cils, les sourcils, les ongles et les cheveux.

Cette composition peut se présenter notamment sous forme de produit coulé en stick ou en coupelle comme les rouges ou baumes à lèvres, les fonds de teint coulés, les produits anti-cemes, les fards à paupières ou à joues, sous forme de pâte ou de crème plus ou moins fluide comme les fonds de teint ou rouges à lèvres fluides, les eyes liners, les mascaras, les compositions de protection solaire ou de coloration de la peau ou encore de maquillage du corps.

Les produits de maquillage ou de soin de la peau ou des lèvres des êtres humains comme les fonds de teint ou les rouges à lèvres contiennent généralement des phases grasses telles que des cires et des huiles, des pigments et/ou charges et éventuellement des additifs comme des actifs cosmétiques ou dermatologiques. Elles peuvent aussi contenir des produits dits "pâteux", de consistance souple, permettant d'obtenir des pâtes, colorées ou non, à appliquer au pinceau.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

De plus, ces compositions ont tendance à migrer, c'est-à-dire à se propager à l'intérieur des rides et des ridules de la peau qui entourent les lèvres et les yeux, entraînant un effet inesthétique.

Dans la demande JP-A-61-65809 la société Shiseido a décrit des compositions de rouge à lèvres "sans transfert" contenant une résine siloxysilicate (à réseau tridimensionnel), une huile de silicone volatile à chaîne silicone cyclique et des charges pulvérulentes. Par ailleurs, la société Noevier à décrit dans le document JP-A-62-61911 des compositions de rouge à lèvres, d'eye liner et de fonds de teint "sans transfert" comportant une ou plusieurs silicones volatiles associées à une ou plusieurs cires hydrocarbonées.

Ces compositions, bien que présentant des propriétés de "sans transfert" améliorées ont l'inconvénient de laisser sur les lèvres, après évaporation des huiles de silicone, un film qui devient inconfortable au cours du temps (sensation de dessèchement et de tiraillement), écartant un certain nombre de femmes de ce type de rouge à lèvres.

Plus récemment, la société Procter & Gambie a envisagé dans sa demande de brevet WO-A-96/36323 des compositions de mascara de type émulsion eau-dans-huile présentant une longue tenue, une résistance à l'eau et ne laissant pas de traces. Ces compositions contiennent, entre autre, un polymère insoluble dans l'eau, appelé généralement un latex, associé à un tensioactif du type alkyle ou alcoxy diméthicone copolyol, des huiles hydrocarbonées, des pigments et charges ainsi que des cires.

Les compositions à base d'huiles de silicones et de résines siliconées ainsi que celles à base de latex conduisent à des films colorés mats. Or, la femme est aujourd'hui à la recherche de produits notamment de coloration des lèvres, brillants. De plus, les propriétés de sans transfert des films déposés ne sont pas parfaites. En particulier, une pression ou un frottement prononcé, conduit à une diminution de la couleur du dépôt et à un redépôt sur le support mis en contact avec ces films.

En outre, les documents EP-A-497144 et FR-A-2 357 244 décrivent des compositions dites "sans transfert", contenant un polymère bloc styrène-éthylène-propylène associé à des cires, des huiles légères ou volatiles et des pigments. Ces compositions présentent l'inconvénient d'être peu confortables, d'avoir des propriétés cosmétiques quelconques et d'être difficilement formulables. Par ailleurs, les propriétés "sans transfert" de ces compositions sont très moyennes.

Il subsiste donc le besoin d'une composition ne présentant pas les inconvénients ci-dessus, et ayant notamment des propriétés de "sans transfert" total, même lors d'une pression ou d'un frottement prononcé, un aspect plus ou moins brillant, adapté au désir de la consommatrice, ne migrant pas, ne desséchant pas la peau ou les lèvres sur lesquelles elle est appliquée, aussi bien lors de l'application qu'au cours du temps.

La demanderesse a constaté, de façon tout à fait surprenante, que l'utilisation d'un polymère dispersible dans une phase grasse, associé à un agent rhéologique particulier, dans une composition cosmétique, dermatologique, pharmaceutique ou hygiénique permettait d'obtenir un film brillant, de très bonne tenue, ne transférant peu ou pas du tout, ne migrant pas, résistant à l'eau, tout en étant très agréable à l'application et à porter tout au long de la journée. Le film est notamment souple et flexible.

La présente invention a donc pour objet une composition à application topique, contenant une phase grasse liquide et des particules de polymère dispersées dans la phase grasse liquide et stabilisées en surface par un agent stabilisant, ladite phase grasse étant, en outre, épaissie par un agent rhéologique liposoluble résultant de la polymérisation d'au moins un monomère à liaison éthylénique.

Par "monomère à liaison éthylénique", on doit comprendre un monomère comportant une ou plusieurs liaisons éthyléniques, éventuellement conjuguées.

Cette composition est en particulier une composition cosmétique, dermatologique, hygiénique ou pharmaceutique. Elle contient donc des ingrédients compatibles avec la peau, les muqueuses et les fibres kératiniques ou phanères. Elle peut se présenter sous forme de gel anhydre, d'émulsion ou de dispersion huile-dans-eau ou eau-dans-huile ou encore sous forme d'émulsion multiple.

Le polymère utilisé dans la présente invention peut être de toute nature. On peut ainsi employer un polymère radicalaire, un polycondensat, voire un polymère d'origine naturelle et leurs mélanges. Le polymère peut être choisi par l'homme du métier en fonction de ses propriétés et selon l'application ultérieure souhaitée pour la composition. De préférence le polymère utilisé est filmifiable. Il est toutefois possible d'utiliser un polymère non filmifiable.

Par polymère non filmifiable, on entend un polymère non capable de former, seul, un film isolable. Ce polymère permet, en association avec un composé non volatil du type huile, de former un dépôt continue et homogène sur la peau et les muqueuses.

Avantageusement, la composition contient au moins un ingrédient choisi parmi les actifs cosmétiques, dermatologiques, hygiéniques et pharmaceutiques, les matières colorantes et leurs mélanges. Grâce à la dispersion de particules de polymère stabilisées en surface présente dans la phase grasse liquide, la composition de l'invention permet de limiter, voire supprimer le transfert de la composition et en particulier le transfert des actifs et/ou des matières colorantes et donc de maintenir ces actifs et/ou matières colorantes là où ils ont été déposés.

Selon l'invention, la quantité de polymère doit être suffisante pour former sur la peau et/ou les lèvres et/ou les fibres kératiniques un film apte à piéger les matières colorantes et/ou les actifs cosmétiques ou dermatologiques et/ou les huiles en vue de limiter, voire supprimer, leur transfert sur un support avec lequel le film est mis en contact. La quantité de polymère est fonction de la quantité de matières colorantes et/ou d'actifs et/ou d'huiles, contenue dans la composition. En pratique, la quantité de polymère peut être supérieure à 2 % en poids (en matière active), par rapport au poids total de la composition.

Un autre objet de l'invention est l'utilisation dans une composition à application topique, cosmétique ou hygiénique ou pour la fabrication d'une composition à application topique, dermatologique ou pharmaceutique, de particules d'au moins un polymère dispersées dans une phase grasse liquide et stabilisées en surface par un agent stabilisant, ladite phase grasse étant épaissie par un agent rhéologique liposoluble résultant de la polymérisation d'au moins un monomère à liaison éthylénique, pour diminuer, voire supprimer, le transfert du film de composition déposé sur les lèvres et/ou la peau d'être humain vers un support mis en contact avec le film et/ou conserver sa brillance.

L'invention a encore pour objet un procédé de soin cosmétique ou de maquillage des lèvres, des phanères ou de la peau, consistant à appliquer sur respectivement les lèvres, les phanères ou la peau une composition cosmétique telle définie précédemment.

L'invention a encore pour objet un procédé pour limiter, voire supprimer, le transfert d'une composition de maquillage ou de soin de la peau ou des lèvres sur un support différent de la dite peau et desdites lèvres, contenant une phase grasse liquide et au moins un ingrédient choisi parmi les matières colorantes et les actifs cosmétiques, dermatologiques, hygiéniques et pharmaceutiques, consistant à introduire dans la phase grasse liquide des particules de polymère dispersibles dans la phase grasse liquide et stabilisables en surface par un agent stabilisant et au moins un agent rhéologique liposoluble résultant de la polymérisation d'au moins un monomère à liaison éthylénique.

Un avantage de l'utilisation d'une dispersion de particules dans une composition de l'invention est que les particules restent à l'état de particules élémentaires, sans former d'agglomérats, dans la phase grasse, ce qui ne serait pas le cas avec des particules minérales de taille nanométrique. Un autre avantage de la dispersion de polymère est la possibilité d'obtenir des compositions très fluides (de l'ordre de 130 centipoises), même en présence d'un taux élevé de polymère.

Encore un autre avantage d'une telle dispersion est qu'il est possible de calibrer à volonté la taille des particules de polymère, et de moduler leur "polydispersité" en taille lors de la synthèse. Il est ainsi possible d'obtenir des particules de très petite taille, qui sont invisibles à l'oeil nu lorsqu'elles sont dans la composition et lorsqu'elles sont appliquées sur la peau ou les lèvres. Ceci ne serait pas possible avec des pigments sous forme particulaire, leur constitution ne permettant pas de moduler la taille moyenne des particules.

On a de plus constaté que la composition selon l'invention, présente des qualités d'étalement et d'adhésion sur la peau, les semi-muqueuses ou les muqueuses, particulièrement intéressantes, ainsi qu'un toucher onctueux et agréable. Cette composition a, en outre, l'avantage de se démaquiller facilement notamment avec un lait démaquillant classique. Ceci est tout à fait remarquable puisque les compositions de l'art antérieur à propriétés "sans transfert" élevées sont très difficiles à démaquiller. En général, elles sont vendues avec un produit démaquillant spécifique, ce qui introduit une contrainte supplémentaire pour l'utilisatrice.

La composition selon l'invention comprend donc avantageusement une ou plusieurs dispersions stables de particules généralement sphériques d'un ou plusieurs polymères, dans une phase grasse liquide physiologiquement acceptable. Ces dispersions peuvent notamment se présenter sous forme de nanoparticules de polymères en dispersion stable dans ladite phase grasse. Les nanoparticules sont de préférence d'une taille comprise entre 5 et 600 nm, étant donné qu'au-delà d'environ 600 nm, les dispersions de particules deviennent beaucoup moins stables.

Encore un avantage de la dispersion de polymère de la composition de l'invention est la possibilité de faire varier la température de transition vitreuse (Tg) du polymère ou du système polymérique (polymère plus additif du type plastifiant), et de passer ainsi d'un polymère dur à un polymère plus ou moins mou, permettant de régler les propriétés mécaniques de la composition en fonction de l'application envisagée.

Il est possible d'utiliser des polymères filmifiables, de préférence ayant une Tg basse, inférieure ou égale à la température de la peau, notamment inférieure à 40°C. On obtient ainsi une dispersion qui peut filmifier lorsqu'elle est appliquée sur un support, ce qui n'est pas le cas lorsqu'on utilise des dispersions de pigments minéraux selon l'art antérieur.

Les polymères utilisables dans la composition de l'invention ont de préférence un poids moléculaire de l'ordre de 2000 à 10 000 000 et une Tg de -100°C à 300°C et mieux de -10° à 50°C.

Lorsque le polymère présente une température de transition vitreuse trop élevée pour l'application souhaitée, on peut lui associer un plastifiant de manière à abaisser cette température du mélange utilisé. Le plastifiant peut être choisi parmi les plastifiants usuellement utilisés dans le domaine d'application et notamment parmi les composés susceptibles d'être des solvants du polymère.

Parmi les polymères filmifiables, on peut citer des homopolymères ou des copolymères radicalaires, acryliques ou vinyliques, de préférence ayant une Tg inférieure ou égale à 40°C et notamment allant de -10° à 30°C.

Parmi les polymères non filmifiables, on peut citer des homopolymères ou copolymères radicalaires, vinyliques ou acryliques, éventuellement réticulés, ayant de préférence une Tg supérieure ou égale à 40°C et notamment allant de 45° à 150°C.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés $\alpha,\beta$-éthyléniques tes que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), comme les (méth)acrylates d'alkyle, en particulier d'alkyle en $C_1$-$C_{20}$, de préférence en $C_1$-$C_8$, les (méth)acrylates d'aryle, en particulier d'aryle en $C_6$-$C_{10}$, les (méth)acrylates d'hydroxyalkyle, en particulier d'hydroxyalkyle en $C_2$-$C_6$. Comme (méth)acrylates d'alkyle, on peut citer le (méth)acrylate de méthyle, d'éthyle, de butyle, d'isobutyle, d'éthyl-2 hexyle et le lauryle. Comme (méth)acrylates d'hydroxyalkyle, on peut citer le (méth)acrylate d'hydroxyéthyle, le (méth)acrylate de 2-hydroxypropyle. Comme (méth)acrylates d'aryle, on peut citer l'acrylate de benzyle ou de phényle.

Les esters de l'acide (méth)acrylique particulièrement préférés sont les (méth)acrylates d'alkyle.

Comme polymère radicalaire, on utilise de préférence les copolymère d'acide (méth)acrylique et de (méth) acrylate d'alkyle, notamment d'alkyle en $C_1$-$C_4$. Plus préférentiellement, on peut utiliser les acrylates de méthyle éventuellement copolymérisés avec l'acide acrylique.

Comme amides des monomères acides, on peut citer les (méth)acrylamides, et notamment les N-alkyl (méth) acrylamides, en particulier d'alkyl en $C_2$-$C_{12}$ tels que le N-éthyl acrylamide, le N-t-butyl acrylamide, le N-octyl acrylamide ; les N- dialkyl ($C_1$-$C_4$) (méth)acrylamides.

Les polymères vinyliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyl, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle. Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques, on peut encore citer :

- la N-vinylpyrrolidone, la vinylcaprolactame, les vinyl N-alkyl($C_1$-$C_6$) pyrroles, les vinyloxazoles, les vinyl-thiazoles, les vinylpyrimidines, les vinylimidazoles,
- les oléfines tels que l'éthylène, le propylène, le butylène, l'isoprène, le butadiène.

Le polymère vinylique peut être réticulé à l'aide de monomère difonctionnel, notamment comprenant au moins deux insaturations éthyléniques, tel que le diméthacrylate d'éthylène glycol ou le phtalate de diallyle.

De façon non limitative, les polymères de l'invention peuvent être choisis parmi, les polymères ou copolymères suivants : polyuréthannes, polyuréthannes-acryliques, polyurées, polyurée-polyuréthannes, polyester-polyuréthannes, polyéther-polyuréthannes, polyesters, polyesters amides, polyesters à chaîne grasse, alkydes polymères ou copolymères acryliques et/ou vinyliques ; copolymères acryliques-silicone polyacrylamides ; polymères siliconés comme les polyuréthannes ou acryliques siliconés, polymères fluorés et leurs mélanges.

La phase grasse liquide de la composition peut être constituée de toute huile cosmétiquement ou dermatologiquement acceptable, et de façon générale physiologiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

Par "phase grasse liquide", on entend tout milieu non aqueux liquide à température ambiante (25°C) et pression atmosphérique. Cette phase grasse peut contenir une phase grasse liquide volatile et/ou une phase grasse non volatile.

Par "phase grasse volatile", on entend tout milieu non aqueux susceptible de s'évaporer de la peau ou des lèvres, en moins d'une heure. Cette phase volatile comporte notamment des huiles ayant une pression de vapeur, à température ambiante et pression atmosphérique allant de $10^{-3}$ à 300mm de Hg (0,13 Pa à 40 000 Pa).

La phase grasse liquide totale de la composition peut représenter de 5 % à 97,90 % du poids total de la composition et de préférence de 20 à 85 %. La partie non volatile peut représenter de 0 à 80 % du poids total de la composition et mieux de 1 à 50%.

Comme phase grasse liquide utilisable dans l'invention, on peut ainsi citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les polydiméthylsiloxanes (PDMS), éventuellement phénylées telles que les phényltriméthicones ou éventuellement substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées.

Avantageusement, on peut utiliser une ou plusieurs huiles volatiles à température ambiante. Ces huiles volatiles sont favorables à l'obtention d'un film à propriétés "sans transfert" total. Après évaporation de ces huiles, on obtient un dépôt filmogène souple, non collant sur la peau ou les muqueuses, suivant respectivement le mouvements de la peau ou des lèvres, sur lesquelles la composition est appliquée. Ces huiles volatiles facilitent, en outre, l'application de la composition sur la peau, les muqueuses et les phanères. Elles peuvent être hydrocarbonées ou siliconées comportant éventuellement des groupements alkyle ou alkoxy, pendants ou en bout de chaîne siliconée.

Comme huile volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone ainsi que les isoparaffines en $C_8$-$C_{16}$. Ces huiles volatiles représentent notamment de 20 à 97,90 % du poids total de la composition, et mieux de 30 à 75 %.

Comme huile volatile utilisable dans l'invention, on peut citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane ou les isoparaffines en $C_8$-$C_{16}$ telles que les 'ISOPARs', les PERMETYLs et notamment l'isododécane.

Dans un mode particulier de réalisation de l'invention, on choisit le phase grasse liquide dans le groupe comprenant :

- les composés liquides non aqueux ayant un paramètre de solubilité global selon l'espace de solubilité de HANSEN inférieur à 17 $(MPa)^{\frac{1}{2}}$,
- ou les monoalcools ayant un paramètre de solubilité global selon l'espace de solubilité de HANSEN inférieur ou égal à 20 $(MPa)^{\frac{1}{2}}$,
- ou leurs mélanges.

Le paramètre de solubilité global δ global selon l'espace de solubilité de HANSEN est défini dans l'article "Solubility parameter values" de Eric A. Grulke de l'ouvrage "Polymer Handbook" 3ème édition, Chapitre VII, pages 519-559 par la relation :

$$\delta = (d_D{}^2\ d_P{}^2 + d_H{}^2)^{\frac{1}{2}}\ ,$$

dans laquelle :

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.). La définition des solvants dans l'espace de solubilité tridimensionnel selon HANSEN est donnée dans l'article de C. M. HANSEN : "The three dimensional solubility parameters" J. Paint Technol. 39, 105 (1967).

Parmi les phases grasses liquides ayant un paramètre de solubilité global selon l'espace de solubilité de HANSEN, inférieur ou égal à 17 $(MPa)^{\frac{1}{2}}$, on peut citer des huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle. On peut également citer les hydrocarbures et notamment des huiles de paraffine, de vaseline, ou le polyisobutylène hydrogéné, l'isododécane, ou encore les 'ISOPARs', isoparaffines volatiles. On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyle, thiol et/ou amine, et les huiles siliconées volatiles, notamment cycliques. On peut également citer les solvants, seuls ou en mélange, choisis parmi (i) les esters linéaires, ramifiés ou cycliques, ayant plus de 6 atomes de carbone, (ii) les éthers ayant plus de 6 atomes de carbone, (iii) les cétones ayant plus de 6 atomes de carbone. Par monoalcools ayant un paramètre de solubilité global selon l'espace de solubilité de HANSEN inférieur ou égal à 20 $(MPa)^{\frac{1}{2}}$, on entend les alcools gras aliphatiques ayant au moins 6 atomes de carbone, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

Comme milieu non aqueux, on peut aussi utiliser ceux décrits dans le document FR-A-2 710 646 de L.V.M.H.

Le choix du milieu non aqueux est effectué par l'homme du métier en fonction de la nature des monomères constituant le polymère et/ou de la nature du stabilisant, comme indiqué ci-après.

La dispersion de polymère peut être fabriquée comme décrit dans le document EP-A-749747. La polymérisation peut être effectuée en dispersion, c'est-à-dire par précipitation du polymère en cours de formation, avec protection des particules formées avec un stabilisant.

On prépare donc un mélange comprenant les monomères initiaux ainsi qu'un amorceur radicalaire. Ce mélange est dissous dans un solvant appelé, dans la suite de la présente description, "solvant de synthèse". Lorsque la phase grasse est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc un solvant de synthèse tel que les monomères initiaux, et l'amorceur radicalaire, y sont solubles, et les particules de polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque la phase grasse choisie est une huile volatile, on peut directement effectuer la polymérisation dans ladite huile qui joue donc également le rôle de solvant de synthèse. Les monomères doivent également y être solubles, ainsi que l'amorceur radicalaire, et le polymère obtenu doit y être insoluble.

Les monomères sont de préférence présents dans le solvant de synthèse, avant polymérisation, à raison de 5-20% en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

*Stabilisant*

Les particules de polymère sont stabilisées en surface, au fur et à mesure de la polymérisation, grâce à un stabilisant qui peut être un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide poly-12(hydroxystéarique).

Ainsi, on peut utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

On peut aussi utiliser des copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou des alkyl ($C_2$-$C_{18}$) diméthicones copolyol tels que ceux vendu sous la dénomination "DOW CORNING 3225C" par la société DOW CORNING, les lauryl méthicones tels que ceux vendu sous la dénomination "DOW CORNING Q2-5200 par la société "DOW CORNING".

Comme copolymères blocs greffés ou séquencés, on peut citer ceux comprenant au moins un bloc résultant de la polymérisation d'au moins un monomère éthylénique, à une ou plusieurs liaisons éthyléniques éventuellement conjuguées comme l'éthylène ou les diènes tels que le butadiène et l'isoprène, et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces polymères, on peut citer les copolymères séquencés, notamment de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène) (voir ci-après les noms commerciaux de l'agent rhéologique). Ces polymères sont généralement appelés des copolymères de diène hydrogéné ou non.

Comme copolymères blocs greffés ou séquencés comprenant au moins un bloc résultant de la polymérisation d'au moins un monomère éthylénique à une ou plusieurs liaisons éthyléniques et d'au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique à une ou plusieurs liaisons éthyléniques et d'au moins un bloc d'un polyéther, tel qu'un polyalkylène en $C_2$-$C_{18}$ (polyoxéthyléné et/ou polyoxypropyléné notamment), on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le solvant de synthèse envisagé.

On peut ainsi employer des copolymères à base d'acrylates ou de méthacrylates d'alkyle issus d'alcools en $C_1$-$C_4$, et d'acrylates ou de méthacrylates d'alkyle issus d'alcools en $C_8$-$C_{30}$. On peut en particulier citer le copolymère méthacrylate de stéaryle/méthacrylate de méthyle.

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

Lorsque le phase grasse liquide ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyle issus d'alcools en $C_1$-$C_4$, et d'acrylates ou de méthacrylates d'alkyle issus d'alcools en $C_8$-$C_{30}$,
- (c) les copolymères blocs greffés ou séquencés comprenant au moins un bloc résultant de la polymérisation d'au moins un monomère éthylénique, à liaisons éthyléniques conjuguées, et au moins un bloc d'un polymère vinylique ou acrylique ou d'un polyéther ou d'un polyester, ou leurs mélanges.

De préférence, on utilise des polymères dibloc comme agent stabilisant.

*Agent rhéologique*

Selon l'invention, il est possible d'utiliser un ou plusieurs agents rhéologiques liposolubles dans la composition de l'invention. Ce ou ces agents rhéologiques sont des agents liposolubles capables d'épaissir et/ou gélifier la composition. Il sont en particulier présents en une quantité efficace pour augmenter la viscosité de la composition jusqu'à l'obtention d'un gel, à savoir un produit ne s'écoulant pas sous son propre poids, voire même un stick. La quantité d'agent rhéologique est fonction de la viscosité recherchée pour la composition finale. En pratique, l'agent rhéologique ou le mélange d'agents rhéologiques représente de 0,05 à 20 % du poids total de la composition, de préférence de 0,1 % à 10 % en poids et mieux de 0,5 à 5 %.

Les agents rhéologiques selon l'invention résultent chacun de la polymérisation ou copolymérisation d'un monomère éthylénique, comportant une ou plusieurs liaisons éthyléniques, de préférence conjuguées (ou diène). Ce ou ces agents sont en particuliers des copolymères vinyliques, acryliques ou méthacryliques qui peuvent être séquencés et notamment de type dibloc ou tribloc, voire même de type multibloc ou en étoile.

Le ou les agents rhéologiques éthyléniques selon l'invention comprennent de préférence un bloc styrénique (S), un bloc alkylstyrénique (AS), un bloc éthylène/butylène (EB), un bloc éthylène/propylène (EP), un bloc butadiène (B), un bloc isoprène (I), un bloc acrylate (A), un bloc méthacrylate (MA) ou une association de ces blocs.

En particulier, on utilise comme agent rhéologique un copolymère comportant au moins un bloc styrénique. De préférence, on utilise un copolymère tribloc et en particulier ceux du type polystyrène/polyisoprène ou polystyrène/polybutadiène tels que ceux vendus sous le nom de 'LUVITOL HSB' par BASF et ceux du type polystyrène/copoly (éthylènepropylène) ou encore du type polystyrène/copoly(éthylène-butylène) tels que ceux vendus sous la marque 'KRATON' par Shell Chemical Co ou de Gelled Permethyl 99A par Penreco. On peut aussi utiliser des copolymères styrène-méthacrylate.

Comme agent rhéologique utilisable dans la composition de l'invention, on peut citer par exemple le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton G1750X (EP) multibras, le Kraton G1765X (EP) multibras, le Kraton D-1101 (SBS), le Kraton D-1102 (SBS), le Kraton D-1107 (SiS), le Gelled Permethyl 99A -750, Gelled Permethyl 99A- 753-58 (mélange de tribloc et de polymère bloc en étoile), Gelled Permethyl 99A- 753-59 (mélange de tribloc et de polymère bloc en étoile), Versagel 5970 et Versagel 5960 de chez Penreco (mélange de tribloc et de polymère en étoile dans isododécane), OS 129880, OS 129881 et OS 84383 de chez Lubrizol (copolymère styrène-méthacrylate).

Ce ou ces agents rhéologiques liposolubles permettent un épaississement de la composition, tout en conservant un aspect brillant à la composition ainsi qu'au film déposé sur les lèvres et/ou le corps. Ceci ressort clairement du tableau (I) mentionné ultérieurement

*Additifs*

Les dispersions de polymères gélifiées obtenues selon l'invention peuvent alors être utilisées dans une composition notamment cosmétique, dermatologique, pharmaceutique et/ou hygiénique, telle qu'une composition de soin ou de maquillage de la peau ou des lèvres, ou encore une composition capillaire ou une composition solaire ou de coloration ou de bronzage artificiel de la peau.

Suivant l'application, on pourra choisir d'utiliser des dispersions de polymères filmifiables ou non filmifiables, dans des huiles volatiles ou non volatiles.

La composition de l'invention peut comprendre avantageusement une ou plusieurs matières colorantes contenant un ou plusieurs composés pulvérulents et/ou un ou plusieurs colorants liposolubles ou hydrosolubles, par exemple à raison de 0 à 70% du poids total de la composition et notamment de 0,01 à 70%. Les composés pulvérulents peuvent être choisis parmi les pigments et/ou les nacres et/ou les charges habituellement utilisés dans les compositions cosmétiques ou dermatologiques. Avantageusement, les composés pulvérulents représentent de 0 à 50 % et notamment de 0,1 à 50 % du poids total de la composition et mieux de 1 à 40 %. Plus la quantité de composés pulvérulents diminue, plus les qualités de sans transfert et de confort augmentent. Le fait que les propriétés de sans transfert augmentent au fur et à mesure que la quantité de composés pulvérulents diminue est tout à fait surprenant. En effet, jusqu'à ce jour les propriétés de sans transfert des compositions de l'art antérieur augmentaient avec la quantité de composés pulvérulents. Inversement, leurs inconforts, leurs brillance et leur sécheresse sur la peau ou muqueuses augmentaient.

Par ailleurs, la propriété de sans transfert augmente avec la quantité de polymère dispersible dans la phase grasse liquide. En pratique, le polymère peut représenter en matière active jusqu'à 60 % (en matière active ou sèche) du poids total de la composition. En utilisant au-dessus de 12 % en poids de matière active de polymère et d'huile non volatile dans la composition et jusqu'à 60 %, on obtient un film sans transfert total. Entre 2 % et 12 % l'effet sans transfert est notable sans toutefois être total. On peut donc adapter les propriétés sans transfert à volonté, ce qui n'était pas possible avec les compositions sans transfert de l'art antérieur, sans nuire au confort du film déposé.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon (Orgasol de chez Atochem), de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter de 0 à 20 % et notamment 0,01 à 20 % du poids de la compositions et mieux de 0,1 à 6 %. Les colorants hydrosolubles sont par exemple le jus de betterave, le bleu de méthylène et peuvent représenter jusqu'à 6 % du poids total de la composition.

La composition de l'invention peut, en outre, contenir un ou plusieurs actifs cosmétiques ou dermatologiques tels que ceux classiquement utilisés.

Comme actifs cosmétiques, dermatologiques, hygiéniques ou pharmaceutiques, utilisables dans la composition de l'invention, on peut citer les hydratants, vitamines, acides gras essentiels, sphingolipides, filtres solaires. Ces actifs sont utilisés en quantité habituelle pour l'homme et notamment à des concentrations de 0 à 20 % et notamment de 0,001 à 20 % du poids total de la composition.

Le polymère de la composition de l'invention permet la formation d'un film sur la peau, les lèvres et/ou les muqueuses, formant un réseau piégeant les matières colorantes (y compris les charges) et/ou les actifs. Selon la quantité relative de matières colorantes, utilisée par rapport à la quantité de polymère stabilisé, utilisée, il est possible d'obtenir un film plus ou moins brillant et plus ou moins sans transfert.

La composition selon l'invention peut, de plus, comprendre, selon le type d'application envisagée, les constituants classiquement utilisés dans les domaines considérés, qui sont présents en une quantité appropriée à la forme galénique souhaitée.

En particulier, elle peut comprendre, outre, la phase grasse liquide dans laquelle le polymère est stabilisé des phases grasses additionnelles qui peuvent être choisies parmi les cires, les huiles, les gommes et/ou les corps gras pâteux, d'origine végétale, animale, minérale ou de synthèse, voire siliconés, et leurs mélanges.

Parmi les cires solides à température ambiante, susceptibles d'être présentes dans la composition selon l'invention, on peut citer les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25°C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Microemulsions Theory and Practice", L. M. Prince Ed., Academic Press (1977), pages 21-32. Comme cire liquide à température ambiante, on peut citer l'huile de Jojoba.

Les cires peuvent être présentes à raison de 0-50% en poids dans la composition et mieux de 5 à 20 %, en vue de ne pas trop diminuer la brillance de la composition et du film déposé sur les lèvres et/ou la peau.

La composition peut comprendre, en outre, tout additif usuellement utilisé dans de telles compositions, tel que des épaississants autres que l'agent rhéologique éthylénique, des antioxydants, des parfums, des conservateurs, des tensioactifs, des polymères liposolubles comme les polyalkylènes, notamment le polybutène, les polyacrylates et les polymères siliconés compatibles avec la phase grasse ainsi que les dérivés de polyvinylpyrolidone. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Dans un mode de réalisation particulier de l'invention, les compositions selon l'invention peuvent être préparées de manière usuelle par l'homme du métier. Elles peuvent se présenter sous forme d'un produit coulé et par exemple sous la forme d'un stick ou bâton, ou sous la forme de coupelle utilisable par contact direct ou à l'éponge. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple, de viscosité dynamique à 25°C de l'ordre de 1 à 40 Pa.s ou encore de gel, de crème plus ou moins fluide. Elles peuvent alors constituées des fonds de teint ou des rouges à lèvres, des produits solaires ou de coloration de la peau.

Les compositions de l'invention sont avantageusement anhydres et peuvent contenir moins de 5 % d'eau par rapport au poids total de la composition. Elles peuvent alors se présenter notamment sous forme de gel huileux, de liquide huileux ou huile, de pâte ou de stick ou encore sous forme de dispersion vésiculaire contenant des lipides ioniques et/ou non ioniques. Elles peuvent aussi se présenter sous forme d'une émulsion simple ou multiple à phase continue huileuse ou aqueuse, de dispersion huileuse dans une phase aqueuse grâce à des vésicules contenant des lipides ioniques et/ou non ioniques. Ces formes galéniques sont préparées selon les méthodes usuelles des domaines considérés.

Ces compositions à application topique peuvent constituer notamment une composition cosmétique, dermatologique, hygiénique ou pharmaceutique de protection, de traitement ou de soin pour le visage, pour le cou, pour les mains ou pour le corps (par exemple crème de soin, huile solaire, gel corporel), une composition de maquillage (par exemple gel de maquillage, crème, stick) ou une composition de bronzage artificiel ou de protection de la peau.

L'invention est illustrée plus en détail dans les exemples suivants. Les pourcentages sont des pourcentages en poids.

**Exemple 1 de dispersion de polymère**

On prépare une dispersion de polyméthacrylate de méthyle réticulé par du diméthacrylate d'éthylène glycol, dans de l'isododécane, selon la méthode de l'exemple 2 du document EP-A-749 746, en remplaçant L'ISOPAR L par de l'isododécane. On obtient ainsi une dispersion de particules de polyméthacrylate de méthyle stabilisées en surface dans de l'isododécane par un copolymère dibloc séquencé polystyrène/copoly(éhylène-propylène) vendu sous le nom de KRATON G1701 (Shell), ayant un taux de matière sèche de 19,7% en poids et une taille moyenne des particules de 135 nm (polydispersité : 0,05) et une Tg de 100°C. Ce copolymère est non filmifiable à température ambiante.

**Exemple 2 de dispersion de polymère**

On prépare une dispersion de copolymère non réticulé d'acrylate de méthyle et d'acide acrylique dans un rapport 95/5, dans de l'isododécane, selon la méthode de l'exemple 1 du document EP-A-749 746, en remplaçant l'heptane par de l'isododécane. On obtient ainsi une dispersion de particules de poly(acrylate de méthyle/acide acrylique) stabilisées en surface dans de l'isododécane par un copolymère dibloc séquencé polystyrène/copoly(éhylène-propylène) vendu sous le nom de KRATON G1701 (Shell), ayant un taux de matière sèche de 19 % en poids et une taille moyenne des particules de 165 nm (polydispersité : 0,05) et une Tg de 13°C. Ce copolymère est filmifiable.

**Exemple 3 de dispersion de polymère**

On prépare une dispersion de copolymère non réticulé d'acrylate de méthyle et d'acide acrylique dans un rapport 95/5, dans de l'isododécane, selon la méthode de l'exemple 1 du document EP-A-749 746, en remplaçant l'heptane par de l'isododécane. On obtient ainsi une dispersion de particules de poly(acrylate de méthyle/acide acrylique) stabilisées en surface dans de l'isododécane par un copolymère dibloc séquencé polystyrène/copoly(éhylène-propylène) vendu sous le nom de KRATON G1701 (Shell), ayant un taux de matière sèche de 22,25 % en poids et une taille moyenne des particules de 180 nm (polydispersité : 0,05) et une Tg de 20°C. Ce copolymère est filmifiable.

**Tests comparatifs**

Dans les dispersions des exemples 2 et 3, on a introduit des pigments, et un gélifiant selon l'invention et selon l'art antérieur. On a ensuite mesuré la viscosité à 25°C des compositions obtenues, à l'aide d'un viscosimètre type Brookfield LV tournant à 100 tr/min et équipé d'un mobile LV4 ou LV3 selon la viscosité ainsi que la brillance à l'aide d'un brillancemétre type Byk Gardner.

Les résultats sont donnés dans le tableau (I) ci-après.

|  | Kraton Dibloc | Kraton Dibloc | Cire Carnauba | Bentone 34 | KSG X-21-5432 |
|---|---|---|---|---|---|
| % Dispersion exemple 2 | 87.4 |  | 88.7 | 85.47 | 84.14 |
| % dispersion exemple 3 |  | 92.2 |  |  |  |
| % gélifiant | 1.3 | 0.75 | 2 | 3.92 | 1.5 |
| % pigments | 3 | 3 | 3 | 3 | 4 |
| viscosité (cps) | 1626 mobile LV4 | 1758 mobile LV4 | 1266 mobile LV4 | 3210 mobile LV4 | 285.6 mobile LV3 |
| brillance | 77.2 | 67.1 | 11.4 | 4.7 | 2.7 |

De ce tableau, il ressort clairement que les gélifiants de la composition selon l'invention assurent une brillance élevée des compositions, ce qui n'est pas le cas avec les gélifiants classiques comme les cires, la Bentone 34 de chez Rhéox, le KSG X-21-5432 vendu par la société Shin Etsu qui est un organopolysiloxane réticulé à 27 % en matière active dans du polyméthyltrifluoropropyl diméthylsiloxane. La composition ne contenant pas de gélifiant ni de pigments a une brillance de 84 %.

**Exemple 4 : Eye liner**

On prépare un eye liner sous forme fluide ayant la composition suivante:

.    polyisobutène hydrogéné         0,77 %
.    oxydes de fer noir         15,00 %
.    dispersion de polymère de l'exemple 3         81,40 %
.    polystyrène/poly(éthylène-propylène) *         1,30 %

. copolymère PVP* /eicosène      0,44 %

. phényltriméthicone      0,76 %

. octyldodécanol      0,33 % Cet eye liner est obtenu selon le mode opératoire suivant : solubiliser le Kraton dans la dispersion de polymère à chaud (environ 50°C) pendant 2h puis ajouter les pigments préalablement broyés dans les huiles.

Cet eye liner a été testé par des personnes expertes en cosmétique. Les résultats du test sont donnés ci-après :

. l'application est facile et glissante,

. la couvrance est bonne,

. le résultat du maquillage est satisfaisant ; le reflet est satiné ou satiné/brillant ; le tracé est net à la lisière,

. l'adhérence est bonne.

Le confort est bon pour 2/3 des testrices, lors de l'application et après-séchage. La tenue est jugée bonne par l'ensemble des femmes.

La tenue est jugée bonne, voire même très bonne pour certaines testrices.

**Exemple 5 : Laque à lèvres**

On prépare une laque à lèvres sous forme fluide ayant la composition suivante :

. polyisobutène hydrogéné      2,23 %

. copolymère de PVP/eicosène      1,28 %

. aluminium laque de DC Red      27 2,00 %

. calcium laque de DC Red 7      3,69 %

. DC Red 36      0,95 %

. oxyde de fer noir      0,07 %

. oxyde de fer rouge 2,09 %

. dispersion de l'exemple 3 83,50 %

. Kraton G-1650 V (Shell Chimie) *      1,00 %

. phényltriméthicone      2,23 %

. octyldodécanol      0,96 %

Cette laque à lèvres est très brillante, sans transfert total et non migrante. Elle est obtenue comme dans l'exemple 4.

**Exemple 6 : Rouge à lèvres sans transfert**

On prépare un rouge à lèvres sous forme fluide ayant la composition suivante:

. polyisobutène hydrogéné      0,77%

. oxydes de fer      4,00 %

. dispersion de l'exemple 2      92,20 %

. Kraton G1701 (Shell Chimie)      1,50 %

. copolymère de PVP/eicosène      0,44 %

. phényltriméthicone      0,76 %

. octyldodécanol      0,33 %

Ce rouge à lèvres est préparer comme dans l'exemple 4. Il a ensuite été testé par des experts en utilisant un applicateur avec un embout en mousse. Il se présente sous forme fluide, brillant, doux à l'application, sans transfert et non migrant.

L'application est facile et rapide. La charge sur l'embout mousse est bonne, la texture est fluide, légère. L'applicateur glisse très bien, le tracé se fait d'un seul geste. Le maquillage des lèvres est rapide, ce rouge à lèvres dépose un film homogène, qui ne nécessite pas de repasser par dessus.

* Kraton - G1701 (Shell Chimie)
** PVP - polyvinylpyrrolidone
* polystyrène/poly(éthylène-buthylène)/polystyrène

Le confort est bon à l'application pour 4/5<sup>ème</sup> des testrices.

Le contour est net sur des lèvres non ridées. Ce rouge à lèvres laisse un beau reflet satiné et une bonne couvrance.

Le transfert est faible. Le dépôt est jugé à l'état de « traces » pour 2/5 essais et « nul » pour 3/5 essais. Il est apprécié par une « bise » sur une feuille de papier 2 minutes après le séchage.

### Exemple 7 : Laque à lèvres

On prépare une laque à lèvres sous forme fluide ayant la composition suivante :

- huile de noyau d'abricot          5,00 %
- polyisobutène hydrogéné          0,77 %
- aluminium laque de DC Red 27          0,91 %
- calcium laque de DC Red 7          1,68 %
- DC Red 36          0,43 %
- oxyde de fer noir          0,03 %
- oxyde de fer rouge          0,95 %
- copolymère de PVP/eicosène          0,43 %
- Kraton G1701 (Shell Chimie)          1,30 %
- dispersion de l'exemple 3          87,4 0%
- phényltriméthicone          0,77 %
- octyldodécanol          0,33 %

Cette laque à lèvres est préparée comme dans l'exemple 4 avec ajout de l'huile d'abricot dans la dispersion de polymère avant l'ajout des pigments.

### Exemple 8 : Fond de teint fluide

On prépare un fond de teint sous forme fluide ayant la composition suivante :

- polyisobutène hydrogéné          7,00 %
- oxyde de fer jaune          1,17%
- oxyde de fer noir          0,26 %
- oxyde de fer brun          0,64 %
- dioxyde de titane          7,93 %
- dispersion de polymère de l'exemple 2          55,00 %
- polystyrène/poly(éthylène-propylène)          2,00 %
- nylon-12          12,00 %
- copolymère PVP/eicosène          4,00 %
- phényltriméthicone          7,00 %
- octyldodécanol          3,00 %

Ce fond de teint est préparé comme l'exemple 4. Il se présente sous forme fluide, s'étale bien, est confortable et a de très bonnes propriétés sans transfert.

### Exemple 9 : Rouge à lèvres brillant à propriétés sans transfert améliorées

On prépare un rouge à lèvres sous forme de stick ayant la composition suivante :

- huile de sésame          8,06 %
- hyaluronate de sodium          0,06 %
- conservateur          0,06 %
- huile de lanoline          qsp 100 %
- lanoline acétylée          4,04 %
- huile d'arara          8,06 %
- érucate d'oléyle          8,06 %
- cire microcristalline          6,18 %
- huile de rosier muscat          1,05 %

- dioxyde de titane        1,81 %
- FD & C Yellow N° 6 laque d'aluminium        3,35 %
- oxydes de fer noir        0,06 %
- aluminium laque DC Red 21        0,06 %
- calcium laque DC Red 7        2,88 %
- méthoxycinnamate d'octyle        0,42 %
- hectorite quartemium-18 (Bentone 37 V)        0,35 %
- dispersion de l'exemple 2        31,00 %
- Kraton G1650E (Shell Chimie)        0,60 %
- cire de PPG-5 lanoline        4,03 %
- cire d'abeille polyglycérolée        2,46 %
- octyldodécanol        0,31 %
- acétate de tocophérol        0,31 %

Ce rouge à lèvres se présente sous forme d'un stick. Il a été testé en comparaison avec un rouge à lèvres classique ne contenant pas la dispersion de polymère ni le Kraton G1650E. Ce rouge à lèvres classique est un rouge à lèvres très confortable.

Ces 2 rouges à lèvres ont été reconnu d'application très facile et rapide, de texture souple, onctueuse, glissante et adhérente. La couvrance est supérieure pour le rouge à lèvres de l'invention et le film formé est plus homogène que ceux du rouge à lèvres classique. Le maquillage est jugé satisfaisant, les bords sont nets. Le film est satiné. Le transfert est apprécié en comparatif par demi-lèvres par une bise sur un papier filtre, 2 minutes après l'application. Le transfert est jugé moins important (léger à moyen) avec le rouge à lèvres de l'invention pour toutes les testrices par rapport à celui de l'art antérieur (moyen + à important). Le confort après application est jugé satisfaisant pour les 2 rouges à lèvres.

**Revendications**

1. Composition à application topique contenant une phase grasse liquide et des particules de polymère dispersées dans la phase grasse liquide et stabilisées en surface par un agent stabilisant, ladite phase étant, en outre, épaissie par un agent rhéologique liposoluble résultant de la polymérisation d'au moins un monomère à liaison éthylénique.

2. Composition selon la revendication 1, dans laquelle l'agent rhéologique est un polymère vinylique, acrylique ou méthacrylique.

3. Composition selon la revendication 1 ou 2, dans laquelle l'agent rhéologique est un copolymère bloc.

4. Composition selon l'une des revendications précédentes, dans laquelle l'agent rhéologique est un copolymère comportant au moins un bloc styrénique et au moins un bloc éthylène-propylène ou éthylène-buthylène ou méthacrylate.

5. Composition selon l'une des revendications précédentes, dans laquelle l'agent rhéologique est un copolymère tribloc.

6. Composition selon l'une des revendications précédentes, dans laquelle l'agent rhéologique représente de 0,05 à 20 % du poids total de la composition.

7. Composition selon l'une des revendications précédentes, dans laquelle l'agent rhéologique représente de 0,5 à 5 % du poids total de la composition.

8. Composition selon l'une des revendications précédentes, **caractérisée en ce que** le polymère en dispersion est filmifiable.

9. Composition selon l'une des revendications précédentes, dans laquelle au moins un actif choisi parmi les actifs cosmétiques, dermatologiques, hygiéniques ou pharmaceutiques est prévu.

10. Composition selon l'une des revendications précédentes, comprenant, en outre, au moins une matière colorante.

**11.** Composition selon l'une des revendications précédentes, dans laquelle le polymère en dispersion est choisi parmi les polymères radicalaires, les polycondensats, les polymères d'origine naturelle et leurs mélanges.

**12.** Composition selon l'une des revendications précédentes, dans laquelle le polymère en dispersion est choisi parmi les polyuréthannes, polyuréthannes-acryliques, polyurées, polyurée/polyuréthannes, polyester-polyuréthannes, polyéther-polyuréthannes, polyesters, polyesters amides, polyesters à chaîne grasse, alkydes polymères ou copolymères acryliques et/ou vinyliques ; copolymères acryliques-silicone ; polyacrylamides ; polymères siliconés, polymères fluorés et leurs mélanges.

**13.** Composition selon l'une des revendications précédentes, dans laquelle la phase grasse liquide est constituée d'huiles d'origine minérale, animale, végétale ou synthétique, carbonées, hydrocarbonées, fluorées et/ou siliconées, seules ou en mélange.

**14.** Composition selon l'une des revendications précédentes, dans laquelle la phase grasse liquide est choisie parmi l'huile de paraffine ou de vaseline, l'huile de vison, de tortue, de soja, le perhydrosqualène, l'huile d'amande douce, de calophyllum, de palme, de parléam, de pépins de raisin, de sésame, de maïs, de colza, de tournesol, de coton, d'abricot, de ricin, d'avocat, de jojoba, d'olive ou de germes de céréales ; des esters d'acide lanolique, d'acide oléique, d'acide laurique, d'acide stéarique; les esters gras tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les PDMS éventuellement phénylés tels que les phényltriméthicones ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées ; les huiles volatiles telles que l'octaméthylcyclotétrasiloxane, le décaméthylcyclopentasiloxane, l'hexadéméthylcyclohexasiloxane, l'heptaméthylhexyltrisiloxane, l'heptaméthyloctyltrisiloxane ou les isoparaffines en $C_8$-$C_{16}$, et l'isododécane.

**15.** Composition selon l'une des revendications précédentes, **caractérisée en ce que** la phase grasse contient au moins une huile volatile à température ambiante et pression atmosphérique.

**16.** Composition selon l'une des revendications précédentes, dans laquelle le stabilisant est choisi parmi les polymères séquencés, les polymères greffés, les polymères statistiques et leurs mélanges.

**17.** Composition selon l'une des revendications précédentes, dans laquelle le stabilisant est choisi parmi les polymères siliconés greffés avec une chaîne hydrocarbonée ; les polymères hydrocarbonés greffés avec une chaîne siliconée ; les copolymères greffés ayant un squelette insoluble de type polyacrylique avec des greffons solubles de type acide poly-12(hydroxystéarique) ; les copolymères blocs greffés ou séquencés comprenant au moins un bloc de type polyorganosiloxane et au moins un bloc d'un polymère radicalaire ; les copolymères blocs greffés ou séquencés comprenant au moins un bloc de type polyorganosiloxane et au moins d'un polyéther ; les copolymères d'acrylates ou de méthacrylates d'alkyle en $C_1$-$C_4$, ou d'acrylates ou de méthacrylates d'alkyle en $C_8$-$C_{30}$; les copolymères blocs greffés ou séquencés comprenant au moins un bloc résultant de la polymérisation de monomères éthyléniques comportant éventuellement des liaisons conjuguées et au moins un bloc d'un polymère vinylique ; les copolymères blocs greffés ou séquencés comprenant au moins un bloc résultant de la polymérisation de monomères éthyléniques comportant éventuellement des liaisons conjuguées et au moins un bloc d'un polymère acrylique ; les copolymères blocs greffés ou séquencés comprenant au moins un bloc résultant de la polymérisation de diène et au moins un bloc d'un polyéther.

**18.** Composition selon l'une des revendications précédentes, **caractérisée en ce que** le stabilisant est un polymère bloc greffé ou séquencé, comprenant au moins un bloc résultant de la polymérisation de diène et au moins un bloc d'un polymère vinylique.

**19.** Composition selon l'une des revendications précédentes, comprenant, en outre, au moins une phase grasse additionnelle choisie parmi les cires, les gommes et/ou les corps gras pâteux, d'origine végétale, animale, minérale, de synthèse, ou siliconée, et leurs mélanges.

**20.** Composition selon l'une des revendications 10 à 19, **caractérisée en ce que** la matière colorante comprend au moins un composé pulvérulent choisi parmi les charges, les pigments, les nacres et leurs mélanges.

**21.** Composition selon la revendication 20, **caractérisée en ce que** le composé pulvérulent représente jusqu'à 50 % du poids total de la composition.

**22.** Composition selon l'une des revendications 20 à 21, **caractérisée en ce que** le composé pulvérulent représente de 1 à 40 % du poids total de la composition.

**23.** Composition selon l'une des revendications précédentes, **caractérisée en ce que** le polymère en dispersion représente (en matière sèche) jusqu'à 60 % du poids total de la composition.

**24.** Composition selon l'une des revendications précédentes, **caractérisée en ce que** le polymère en dispersion représente (en matière sèche) de 12 à 60 % du poids total de la composition.

**25.** Composition selon l'une des revendications précédentes, **caractérisée en ce que**. la phase grasse liquide contient au moins une huile choisie parmi les isoparaffines en $C_8$-$C_{16}$, l'isododécane, et les silicones linéaires ou cycliques ayant de 2 à 7 atomes de silicium, ces silicones comportant éventuellement des groupes alkyle ayant de 1 à 10 atomes de carbone, leurs mélanges.

**26.** Composition selon l'une des revendications précédentes, se présentant sous forme d'un stick ou bâton, sous la forme d'une pâte souple de viscosité dynamique à 25°C de 1 à 40 Pa.s, sous forme de coupelle, de gel huileux, de liquide huileux, de dispersion vésiculaire contenant des lipides ioniques et/ou non ioniques, d'émulsion eau-dans-huile ou huile-dans-eau.

**27.** Composition selon l'une des revendications précédentes, se présentant sous forme anhydre.

**28.** Composition selon l'une des revendications précédentes, se présentant sous la forme d'un produit de soin et/ou de maquillage de la peau et/ou des lèvres.

**29.** Composition selon l'une des revendications précédentes, se présentant sous forme d'un fond de teint, d'un fard à joues ou à paupières, d'un rouge à lèvres, d'une base ou baume de soin pour les lèvres, d'un produit anti-cernes, d'un eye liner, d'un mascara.

**30.** Utilisation dans ou pour la fabrication d'une composition à application topique, de particules d'au moins un polymère dispersées dans une phase grasse liquide et stabilisées en surface par un agent stabilisant, ladite phase grasse étant épaissie par un agent rhéologique liposoluble résultant de la polymérisation d'au moins un monomère à liaison éthylénique, pour diminuer, voire supprimer, le transfert du film de composition déposé sur la peau et/ou les lèvres d'être humain vers un support mis en contact avec le film et/ou conserver sa brillance.

**31.** Procédé de soin cosmétique ou de maquillage des lèvres ou de la peau, consistant à appliquer sur respectivement les lèvres ou la peau une composition cosmétique telle définie aux revendications 1 à 29.

**32.** Procédé pour limiter, voire supprimer, le transfert d'une composition de maquillage ou de soin de la peau ou des lèvres sur un support différent de la peau ou des lèvres, contenant une phase grasse liquide et au moins un ingrédient choisi parmi les actifs cosmétiques, dermatologiques, hygiéniques et pharmaceutiques et les matières colorantes, consistant à introduire dans la phase grasse liquide un agent rhéologique liposoluble résultant de la polymérisation d'au moins un monomère éthylénique et des particules de polymère dispersibles dans ladite phase grasse liquide et stabilisables en surface par un agent stabilisant.

**Patentansprüche**

**1.** Zusammensetzung zur topischen Anwendung, die eine flüssige Fettphase und Polymerpartikel enthält, die in der flüssigen Fettphase dispergiert und durch ein Stabilisierungsmittel an der Oberfläche stabilisiert sind, wobei die Fettphase durch ein fettlösliches Rheologiehilfsmittel verdickt ist, das bei der Polymerisation mindestens eines Monomers mit ethylenischer Bindung entsteht.

2. Zusammensetzung nach Anspruch 1, wobei das Rheologiehilfsmittel ein Vinylpolymer, Acrylpolymer oder Methacrylpolymer ist.

3. Zusammensetzung nach Anspruch 1 oder 2, wobei das Rheologiehilfsmittel ein Blockcopolymer ist.

4. Zusammensetzung nach einem der vorhergehenden Ansprüche, wobei das Rheologiehilfsmittel ein Copolymer ist, das mindestens einen Styrolblock und mindestens einen Ethylen-Propylen-Block oder Ethylen-Butylen-Block oder Methacrylatblock enthält.

5. Zusammensetzung nach einem der vorhergehenden Ansprüche, wobei das Rheologiehilfsmittel ein Dreiblock-Copolymer ist.

6. Zusammensetzung nach einem der vorhergehenden Ansprüche, wobei das Rheologiehilfsmittel 0,05 bis 20 % des Gesamtgewichts der Zusammensetzung ausmacht.

7. Zusammensetzung nach einem der vorhergehenden Ansprüche, wobei das Rheologiehilfsmittel 0, 5 bis 5 % des Gesamtgewichts der Zusammensetzung ausmacht.

8. Zusammensetzung nach einem der vorhergehenden Ansprüche, **dadurch gekennzeichnet, dass** das dispergierte Polymer filmbildend ist.

9. Zusammensetzung nach einem der vorhergehenden Ansprüche, wobei mindestens ein Wirkstoff enthalten ist, der unter den kosmetischen, dermatologischen, hygienischen oder pharmazeutischen Wirkstoffen ausgewählt ist.

10. Zusammensetzung nach einem der vorhergehenden Ansprüche, die ferner mindestens ein Farbmittel enthält.

11. Zusammensetzung nach einem der vorhergehenden Ansprüche, wobei das Polymer in Dispersion unter den durch radikalische Polymerisation hergestellten Polymeren, Polykondensaten, Polymeren natürlicher Herkunft und deren Gemischen ausgewählt ist.

12. Zusammensetzung nach einem der vorhergehenden Ansprüche, wobei das dispergierte Polymer unter den Polyurethanen, Polyurethan-Acrylpolymeren, Polyharnstoffen, Polyharnstoff-Polyurethanen, Polyester-Polyurethanen, Polyether-Polyurethanen, Polyestern, Polyesteramiden, Polyestern mit Fettkette und Alkydharzen; Acryl- und/oder Vinylpolymeren oder Acrylund/oder Vinylcopolymeren; Acryl-Silicon-Copolymeren; Polyacrylamiden; Siliconpolymeren, fluorierten Polymeren und deren Gemischen ausgewählt ist.

13. Zusammensetzung nach einem der vorhergehenden Ansprüche, wobei die flüssige Fettphase aus Ölen mineralischen, tierischen, pflanzlichen oder synthetischen Ursprungs, Ölen auf Kohlenstoffbasis, Kohlenwasserstoffölen, fluorierten Ölen und/ oder Siliconölen oder Gemischen dieser Öle besteht.

14. Zusammensetzung nach einem der vorhergehenden Ansprüche, wobei die flüssige Fettphase unter Paraffinöl, Vaselineöl, Nerzöl, Schildkrötenöl, Sojaöl, Perhydrosqualen, Süßmandelöl, Calophyllumöl, Palmöl, Parleam, Traubenkernöl, Sesamöl, Maisöl, Rapsöl, Sonnenblumenöl, Baumwollsamenöl, Aprikosenkernöl, Ricinusöl, Avocadoöl, Jojobaöl, Olivenöl oder Öl von Getreidekeimen; Estern von Lanolinsäure, Ölsäure, Laurinsäure und Stearinsäure; Fettsäureestern, wie Isopropylmyristat, Isopropylpalmitat, Butylstearat, Hexyllaurat, Diisopropyladipat, Isononylisononat, 2-Ethylhexylpalmitat, 2-Hexyldecyllaurat, 2-Octyldecylpalmitat, 2-Octyldodecylmyristat, 2-Octyldodecyllactat, 2-Diethylhexylsuccinat, Diisostearylmalat, Glycerintriisostearat oder Diglycerintriisostearat; höheren Fettsäuren, wie Myristinsäure, Palmitinsäure, Stearinsäure, Behensäure, Ölsäure, Linolsäure, Linolensäure oder Isostearinsäure; höheren Fettalkoholen, wie Cetanol, Stearylalkohol, Oleylalkohol, Linoleylalkohol, Linolenylalkohol, Isostearylalkohol oder Octyldodecanol; Siliconölen, wie PDMS, die gegebenenfalls Phenylgruppen enthalten, beispielsweise Phenyltrimethiconen, oder gegebenenfalls mit aliphatischen und/oder aromatischen Gruppen oder funktionellen Gruppen substituiert sind, wie Hydroxy-, Thiol- und/oder Aminogruppen; mit Fettsäuren, Fettalkoholen oder Polyoxyalkylenen modifizierten Polysiloxanen, fluorierten Siliconen, perfluorierten Ölen und flüchtigen Ölen, wie Octamethylcyclotetrasiloxan, Decamethylcyclopentasiloxan, Hexadecamethylcyclohexasiloxan, Heptamethylhexyltrisiloxan, Heptamethyloctyltrisiloxan oder den $C_{8-16}$-Iosparaffinen und Isododecan, ausgewählt ist.

15. Zusammensetzung nach einem der vorhergehenden Ansprüche, wobei die flüssige Fettphase mindestens ein bei Raumtemperatur und Atmosphärendruck flüchtiges Öl enthält.

**16.** Zusammensetzung nach einem der vorhergehenden Ansprüche, wobei das Stabilisierungsmittel unter den sequentiellen Polymeren, Pfropfpolymeren, statistischen Polymeren und deren Gemischen ausgewählt ist.

**17.** Zusammensetzung nach einem der vorhergehenden Ansprüche, wobei das Stabilisierungsmittel ausgewählt ist unter: den mit einer Kohlenwasserstoffkette gepfropften Siliconpolymeren; den mit einer Siliconkette gepfropften Kohlenwasserstöffpolymeren; den gepfropften Copolymeren, die ein unlösliches Grundgerüst vom Polyacryltyp mit löslichen Pfropfzweigen vom Poly(12-hydroxystearinsäure)-Typ aufweisen; den gepfropften oder sequentiellen Blockcopolymeren, die mindestens einen Block vom Polyorganosiloxantyp und mindestens einen Block eines durch radikalische Polymerisation hergestellten Polymers enthalten; den gepfropften oder sequentiellen Blockcopolymeren, die mindestens einen Block vom Polyorganosiloxantyp und mindestens einen Polyetherblock aufweisen; den Copolymeren von $C_{1-4}$-Alkylacrylaten oder $C_{1-4}$-Alkylmethacrylaten oder $C_{8-30}$-Alkylacrylaten oder $C_{8-30}$-Alkylmethacrylaten; den gepfropften oder sequentiellen Blockcopolymeren, die mindestens einen Block, der bei der Polymerisation von ethylenischen Monomeren, die gegebenenfalls konjugierte Bindungen enthalten, gebildet wird, und mindestens einen Vinylpolymerblock enthalten; den gepfropften oder sequentiellen Blockcopolymeren, die mindestens einen Block, der bei der Polymerisation von ethylenischen Monomeren, die gegebenenfalls konjugierte Bindungen enthalten, gebildet wird, und mindestens einen Acrylpolymerblock enthalten; und den gepfropften oder sequentiellen Blockcopolymeren, die mindesten's einen Block, der bei der Polymerisation eines Diens gebildet wird, und mindestens einen Polyetherblock enthalten.

**18.** Zusammensetzung nach einem der vorhergehenden Ansprüche, **dadurch gekennzeichnet, dass** das Stabilisierungsmittel ein gepfropftes oder sequentielles Blockpolymer ist, das mindestens einen Block, der bei der Polymerisation eines Diens gebildet wird, und mindestens einen Vinylpolymerblock enthält.

**19.** Zusammensetzung nach einem der vorhergehenden Ansprüche, die ferner mindestens eine zusätzliche Fettphase enthält, die unter den Wachsen, Gummen/Gummis und/oder pastösen Fettsubstanzen pflanzlicher, tierischer, mineralischer oder synthetischer Herkunft oder auf Siliconbasis und deren Gemischen ausgewählt ist.

**20.** Zusammensetzung nach einem der Ansprüche 10 bis 19 **dadurch gekennzeichnet, dass** das Farbmittel mindestens eine pulverförmige Verbindung umfasst, die unter den Füllstoffen, Pigmenten, Perlglanzpigmenten oder deren Gemischen ausgewählt ist.

**21.** Zusammensetzung nach Anspruch 20, **dadurch gekennzeichnet, dass** die pulverförmige Verbindung bis zu 50 % des Gesamtgewichts der Zusammensetzung ausmacht.

**22.** Zusammensetzung nach einem der Ansprüche 20 bis 21, **dadurch gekennzeichnet, dass** die pulverförmige Verbindung 1 bis 40 % des Gesamtgewichts der Zusammensetzung ausmacht.

**23.** Zusammensetzung nach einem der vorhergehenden Ansprüche, **dadurch gekennzeichnet, dass** das dispergierte Polymer (als Trockensubstanz) bis zu 60 % des Gesamtgewichts der Zusammensetzung ausmacht.

**24.** Zusammensetzung nach einem der vorhergehenden Ansprüche, **dadurch gekennzeichnet, dass** das dispergierte Polymer (als Trockensubstanz) 12 bis 60 % des Gesamtgewichts der Zusammensetzung ausmacht.

**25.** Zusammensetzung nach einem der vorhergehenden Ansprüche, **dadurch gekennzeichnet, dass** die flüssige Fettphase mindestens ein Öl enthält, das unter den $C_{8-16}$-Isoparaffinen, Isododecan und den linearen oder cyclischen Siliconen mit 2 bis 7 Siliciumatomen, wobei die Silicone gegebenenfalls Alkylgruppen mit 1 bis 10 Kohlenstoffatomen aufweisen, oder deren Gemischen ausgewählt ist.

**26.** Zusammensetzung nach einem der vorhergehenden Ansprüche, die in Form eines Sticks oder Stiftes; in Form einer weichen Paste mit einer dynamischen Viskosität bei 25 °C in der Größenordnung von 1 bis 40 Pa·s; in Form eines Tiegelchens; als öliges Gel; als ölige Flüssigkeit; oder als Vesikeldispersion, die ionische und/oder nichtionische Lipide enthält, als Wasser-in-Öl-Emulsion oder als Öl-in-Wasser-Emulsion vorliegt.

**27.** Zusammensetzung nach einem der vorhergehenden Ansprüche, die in wasserfreier Form vorliegt.

**28.** Zusammensetzung nach einem der vorhergehenden Ansprüche, die in Form eines Produkts zur Pflege und/oder zum Schminken der Haut und/oder der Lippen vorliegt.

**29.** Zusammensetzung nach einem der vorhergehenden Ansprüche, die als Make-up, Wangenrouge, Lidschatten, Lippenstift, pflegende Grundierung oder Pflegebalsam für die Lippen, Produkt zum Abdecken, Eyeliner oder Mascara vorliegt.

**30.** Verwendung von Partikeln mindestens eines Polymers, die in einer flüssigen Fettphase dispergiert und durch mindestens ein Stabilisierungsmittel an der Oberfläche stabilisiert sind, wobei die Fettphase durch ein fettlösliches Rheologiehilfsmittel verdickt ist, das bei der Polymerisation mindestens eines Monomers mit ethylenischer Bindung entsteht, in einer Zusammensetzung zur topischen Anwendung oder für die Herstellung einer Zusammensetzung zur topischen Anwendung, um das Übertragen eines Films der auf der menschlichen Haut und/oder den Lippen abgeschiedenen Zusammensetzung auf eine Oberfläche, mit der der Film in Kontakt kommt, zu vermindern oder zu verhindern und/oder um seinen Glanz zu bewahren.

**31.** Verfahren zur kosmetischen Pflege oder zum Schminken der Lippen oder der Haut, das darin besteht, auf die Lippen und/oder die Haut eine kosmetische Zusammensetzung nach den Ansprüchen 1 bis 29 aufzubringen.

**32.** Kosmetisches Verfahren, um das Übertragen einer kosmetischen Zusammensetzung zum Schminken oder zur Pflege der Haut oder der Lippen auf einen Träger, der von der Haut und den Lippen verschieden ist, zu vermindern oder zu verhindern, wobei die Zusammensetzung eine flüssige Fettphase und mindestens einen Bestandteil enthält, der unter den kosmetischen, dermatologischen, hygienischen oder pharmazeutischen Wirkstoffen und den Farbmitteln ausgewählt ist, das darin besteht, in die flüssige Fettphase ein fettlösliches Rheologiehilfsmittel, das bei der Polymerisation mindestens eines Monomers mit ethylenischer Bindung entsteht, und Polymerpartikel einzuarbeiten, die in der flüssigen Fettphase dispergierbar sind und durch mindestens ein Stabilisierungsmittel an der Oberfläche stabilisiert werden können

## Claims

**1.** Composition for topical application comprising a liquid fatty phase and polymer particles dispersed in the liquid fatty phase and stabilized at the surface by a stabilizing agent, the said phase additionally being thickened by a fat-soluble rheological agent resulting from the polymerization of at least one monomer possessing an ethylenic bond.

**2.** Composition according to Claim 1, in which the rheological agent is a vinyl, acrylic or methacrylic polymer.

**3.** Composition according to Claim 1 or 2, in which the rheological agent is a block copolymer.

**4.** Composition according to one of the preceding claims, in which the rheological agent is a copolymer comprising at least one styrene block and at least one ethylene-propylene or ethylene-butylene or methacrylate block.

**5.** Composition according to one of the preceding claims, in which the rheological agent is a triblock copolymer.

**6.** Composition according to one of the preceding claims, in which the rheological agent represents from 0.05 to 20% of the total weight of the composition.

**7.** Composition according to one of the preceding claims, in which the rheological agent represents from 0.5 to 5% of the total weight of the composition.

**8.** Composition according to one of the preceding claims, **characterized in that** the polymer in dispersion can form a film.

**9.** Composition according to one of the preceding claims, in which at least one active agent chosen from cosmetic, dermatological, hygiene or pharmaceutical active agents is provided.

**10.** Composition according to one of the preceding claims, additionally comprising at least one colouring material.

**11.** Composition according to one of the preceding claims, in which the polymer in dispersion is chosen from radical polymers, polycondensates, polymers of natural origin and their mixtures.

12. Composition according to one of the preceding claims, in which the polymer in dispersion is chosen from polyurethanes, polyurethane-acrylics, polyureas, polyurea-polyurethanes, polyester-polyurethanes, polyether-polyurethanes, polyesters, polyesteramides, polyesters with a fatty chain or alkyds; acrylic and/or vinyl polymers or copolymers; acrylic-silicone copolymers; polyacrylamides; silicone polymers, fluorinated polymers and their mixtures.

13. Composition according to one of the preceding claims, in which the liquid fatty phase is composed of carbon-comprising, hydrocarbon-comprising, fluorinated and/or silicone oils of mineral, animal, plant or synthetic origin, alone or as a mixture.

14. Composition according to one of the preceding claims, in which the liquid fatty phase is chosen from liquid paraffin or liquid petrolatum, mink oil, turtle oil, soybean oil, perhydrosqualene, sweet almond oil, calophyllum oil, palm oil, parleam oil, grape seed oil, sesame oil, maize oil, rapeseed oil, sunflower oil, cottonseed oil, apricot oil, castor oil, avocado oil, jojoba oil, olive oil or cereal germ oil; esters of lanolic acid, of oleic acid, of lauric acid or of stearic acid; fatty esters, such as isopropyl myristate, isopropyl palmitate, butyl stearate, hexyl laurate, diisopropyl adipate, isononyl isononate, 2-ethylhexyl palmitate, 2-hexyldecyl laurate, 2-octyldecyl palmitate, 2-octyldodecyl myristate or lactate, di(2-ethylhexyl) succinate, diisostearyl malate, glyceryl triisostearate or diglyceryl triisostearate; higher fatty acids, such as myristic acid, palmitic acid, stearic acid, behenic acid, oleic acid, linoleic acid, linolenic acid or isostearic acid; higher fatty alcohols, such as cetanol, stearyl alcohol or oleyl alcohol, linoleyl or linolenyl alcohol, isostearyl alcohol or octyldodecanol; silicone oils, such as PDMS, which are optionally phenylated, such as phenyltrimethicones, or which are optionally substituted by aliphatic and/or aromatic groups or by functional groups, such as hydroxyl, thiol and/or amine groups; polysiloxanes modified by fatty acids, fatty alcohols or polyoxyalkylenes, fluorinated silicones or perfluorinated oils; volatile oils, such as octamethylcyclotetrasiloxane, decamethylcyclopentasiloxane, hexadecamethylcyclohexasiloxane, heptamethylhexyltrisiloxane, heptamethyloctyltrisiloxane or $C_8$-$C_{16}$ isoparaffins, and isododecane.

15. Composition according to one of the preceding claims, **characterized in that** the fatty phase comprises at least one oil which is volatile at room temperature and atmospheric pressure.

16. Composition according to one of the preceding claims, in which the stabilizing agent is chosen from sequential polymers, grafted polymers, random polymers and their mixtures.

17. Composition according to one of the preceding claims, in which the stabilizing agent is chosen from silicone polymers grafted with a hydrocarbon-comprising chain; hydrocarbon-comprising polymers grafted with a silicone chain; grafted copolymers having an insoluble backbone of polyacrylic type with soluble grafts of poly(12-hydroxystearic acid) type; grafted or sequential block copolymers comprising at least one block of polyorganosiloxane type and at least one block of a radical polymer; grafted or sequential block copolymers comprising at least one block of polyorganosiloxane type and at least one block of a polyether; copolymers of $C_1$-$C_4$ alkyl acrylates or methacrylates and of $C_8$-$C_{30}$ alkyl acrylates or methacrylates; grafted or sequential block copolymers comprising at least one block resulting from the polymerization of ethylenic monomers optionally comprising conjugated bonds and at least one block of a vinyl polymer; grafted or sequential block copolymers comprising at least one block resulting from the polymerization of ethylenic monomers optionally comprising conjugated bonds and at least one block of an acrylic polymer; or grafted or sequential block copolymers comprising at least one block resulting from the polymerization of dienes and at least one block of a polyether.

18. Composition according to one of the preceding claims, **characterized in that** the stabilizing agent is a grafted or sequential block copolymer comprising at least one block resulting from the polymerization of dienes and at least one block of a vinyl polymer.

19. Composition according to one of the preceding claims, additionally comprising at least one additional fatty phase chosen from waxes, gums and/or pasty fatty substances of plant, animal, mineral, synthetic or silicone origin, and their mixtures.

20. Composition according to one of Claims 10 to 19, **characterized in that** the colouring material comprises at least one pulverulent compound chosen from fillers, pigments, pearlescent agents and their mixtures.

21. Composition according to Claim 20,
**characterized in that** the pulverulent compound represents up to 50% of the total weight of the composition.

**22.** Composition according to either of Claims 20 and 21, **characterized in that** the pulverulent compound represents from 1 to 40% of the total weight of the composition.

**23.** Composition according to one of the preceding claims, **characterized in that** the polymer in dispersion represents (as dry matter) up to 60% of the total weight of the composition.

**24.** Composition according to one of the preceding claims, **characterized in that** the polymer in dispersion represents (as dry matter) from 12 to 60% of the total weight of the composition.

**25.** Composition according to one of the preceding claims, **characterized in that** the liquid fatty phase comprises at least one oil chosen from $C_8$-$C_{16}$ isoparaffins, isododecane and linear or cyclic silicones having from 2 to 7 silicon atoms, these silicones optionally comprising alkyl groups having from 1 to 10 carbon atoms, and their mixtures.

**26.** Composition according to one of the preceding claims which is provided in the form of a stick, in the form of a supple paste with a dynamic viscosity at 25°C of 1 to 40 Pa.s, in the form of a dish, of an oily gel, of an oily liquid, of a vesicular dispersion comprising ionic and/or non-ionic lipids, or of a water-in-oil or oil-in-water emulsion.

**27.** Composition according to one of the preceding claims which is provided in anhydrous form.

**28.** Composition according to one of the preceding claims which is provided in the form of a product for caring for and/or for making up the skin and/or lips.

**29.** Composition according to one of the preceding claims which is provided in the form of a foundation, of a blusher, of an eyeshadow, of a lipstick, of a base or balm for caring for the lips, of a concealer, of an eyeliner or of a mascara.

**30.** Use, in or for the manufacture of a composition for topical application, of particles of at least one polymer which are dispersed in a liquid fatty phase and which are stabilized at the surface by a stabilizing agent, the said fatty phase being thickened by a fat-soluble rheological agent resulting from the polymerization of at least one monomer possessing an ethylenic bond, in order to decrease, indeed even eliminate, the transfer of the composition film deposited on human skin and/or lips to a substrate brought into contact with the film and/or to retain its gloss.

**31.** Process for the cosmetic care of or for making up the lips or skin, which consists in applying, to the lips or skin respectively, a cosmetic composition as defined in Claims 1 to 29.

**32.** Process for limiting, indeed even eliminating, the transfer of a composition for making up or caring for the skin or lips onto a substrate other than the skin or lips, comprising a liquid fatty phase and at least one ingredient chosen from cosmetic, dermatological, hygiene and pharmaceutical active agents and colouring materials, which consists in introducing, into the liquid fatty phase, a fat-soluble rheological agent resulting from the polymerization of at least one ethylenic monomer and polymer particles which are dispersible in the said liquid fatty phase and which can be stabilized at the surface by a stabilizing agent.